# EUROPEAN PATENT APPLICATION

(11) **EP 3 228 307 A1**
(43) Date of publication of application: **11.10.2017**
(21) Application number: 16163916.6
(22) Date of filing: 05.04.2016
(51) Int. Cl.: A61K 9/10

(54) **SOLID DISPERSION COMPRISING OPIOID ANTAGONISTS**

(71) Applicant: SANDOZ AG, 4056 Basel (CH)
(72) Inventor: STEFINOVIC, Marijan, 6250 Kundl (AT); REECE, Hayley, Milton Bridge, EH26 0BE (GB)
(74) Representative: Prüfer & Partner mbB Patentanwälte · Rechtsanwälte

(57) **Abstract**

The present invention relates to a solid dispersion comprising, preferably consisting of, naloxegol salts in amorphous form and at least one pharmaceutically acceptable matrix compound and wherein the matrix compound is
(i) an organic polymer, or
(ii) a silicon-based inorganic adsorbent.

Further, the present invention also relates to a process for preparing a solid dispersion comprising naloxegol in amorphous form and at least one pharmaceutically acceptable matrix compound, as well as to a solid dispersion obtained or obtainable by said process. Further, the present invention relates to a pharmaceutical composition comprising such solid dispersion as well as a pharmaceutical composition for use as µ-opioid antagonists.

## Description

The present invention relates to a solid dispersion comprising naloxegol [(5α,6α)-17-allyl-6-(2,5,8,11,14,17,20-heptaoxadocosan-22-yloxy)-4,5-epoxymorphinan-3,14-diol] or a salt thereof in amorphous form as µ-opioid antagonist and at least one pharmaceutically acceptable matrix compound. Further, the present invention also relates to a process for preparing a solid dispersion comprising naloxegol or a salt thereof in amorphous form and at least one pharmaceutically acceptable matrix compound, as well as to a solid dispersion obtained or obtainable by said process. Further, the present invention relates to a pharmaceutical composition comprising such a solid dispersion as well as a pharmaceutical composition for use in treating or preventing Opioid-induced bowel dysfunction, wherein the pharmaceutical composition comprises such a solid dispersion.

### BACKGROUND OF THE INVENTION

Opioids relieve pain by attaching to 'opioid receptors' in the brain and spinal cord. However, these receptors are also found in the gut and when opioids attach to the gut receptors, they reduce the movement of the gut and can cause constipation.

Naloxegol [(5α,6α)-17-allyl-6-(2,5,8,11,14,17,20-heptaoxadocosan-22-yloxy)-4,5-epoxymorphinan-3,14-diol, also called mPEG₇-O-naloxol], is a peripheral µ-opioid-receptor antagonist. It attaches to a specific type of opioid receptor called the 'µ-opioid receptor' and blocks opioids from binding to these receptors. Naloxegol is derived from naloxone, a well-known substance that is used to block the action of opioids. Naloxegol is a pegylated (polyethylene glycol-modified) derivative of α-naloxol. Specifically, the 5-α-hydroxyl group of α-naloxol is connected via an ether linkage to the free hydroxyl group of a monomethoxy-terminated n=7 oligomer of PEG, thus making naloxegol less able to enter the brain than naloxone, meaning that it can block the µ -opioid receptors in the gut but less so in the brain. By blocking receptors in the gut, naloxegol reduces constipation due to opioids, but does not interfere with their pain relief effects. This theory is described in detailed in WO 2005/058367; WO 2008/057579; inWebster et al., "NKTR-118 Significantly Reverses Opioid-Induced-Constipation" and in Poster 39, of the 20th AAPM Annual Clinical Meeting (Phoenix, AZ), October 10, 2009.

Naloxegol is disclosed in WO 2005/058367 to be useful for opioid-induced bowel dysfunction and constipation as an opioid receptor µ antagonist. The synthesis of naloxegol is described in WO 2005/058367.

However, naloxegol free base is difficult to formulate because it has not been observed to form a crystalline phase, not even when cooled to -60 °C. Instead naloxegol has been observed to exist as a glass with a transition temperature of approximately -45 °C. Furthermore, naloxegol in its free base form can undergo oxidative degradation upon exposure to air.

WO 2012/044243A1 attempted to produce salts of naloxegol suitable for pharmaceutical preparations. WO 2012/044243A1 discloses that several acids were unsuccessfully tested for the preparation of a crystalline naloxegol salt, including adipic, decanoic, fumaric, maleic, malonic, methanesulfonic, oxalic, pamoic, phosphoric and toluenesulfoic acid, to name but a few of those disclosed in tables 1, 2, 3 and 4 of WO 2012/044243A1. They were chosen for scale-up attempts for producing naloxol salts at approximately at 30 mg scale, using solvents including THF, n-hexanes, cyclohexane, ethyl acetate, ether, DCM, IPE, acetonitrile, MeOH, PrCN, butanol, acetone, and mixtures thereof. However in most instances, the mixture of naloxegol and acid in a solvent resulted in a clear solution, sticky matter, gels or a mere precipitate, but not in a free-flowing solid form which could be isolated without deliquescence. Only a crystalline oxalate salt of naloxegol and a crystalline phosphate salt of naloxegol are obtained in WO 2012/044243A1.

Therefore, it is an objective of the present invention to provide naloxegol salts which are free flowing, non-hygroscopic and/or non-deliquescent, and which are suitable for the preparation of a pharmaceutical composition.

### SUMMARY OF THE INVENTION

Surprisingly, it was found that one or more of these objectives can be solved by providing a solid dispersion comprising naloxegol salts in amorphous form and at least one pharmaceutically acceptable matrix compound, and wherein the at least one matrix compound is
(i) an organic polymer, or
(ii) a silicon-based inorganic adsorbent.

Furthermore, the present invention provides a process for preparing a solid dispersion comprising a naloxegol salt in amorphous form and at least one pharmaceutical acceptable matrix compound, the process comprising
a) providing naloxegol in a solvent,
b) bringing the solution or suspension provided in a step a) into contact with a suitable salt-forming acid,
c) bringing the solution or suspension obtained from step b) into contact with the at least one acceptable matrix compound,
d) removing at least part of the solvent to provide the solid dispersion comprising a naloxegol salt and at least one pharmaceutical acceptable matrix compound.

The present invention further relates to a solid dispersion obtained or obtainable by the above-described processes as well as to a pharmaceutical composition comprising a solid dispersion as described above, or a solid dispersion obtained or obtainable by the above-described processes.

### DETAILED DESCRIPTION

In a first aspect, the present invention provides a solid dispersion comprising a naloxegol salt [(5α,6α)-17-allyl-6-(2,5,8,11,14,17,20-heptaoxadocosan-22-yloxy)-4,5-epoxymorphinan-3,14-diol] in amorphous form and at least one pharmaceutically acceptable solid matrix compound, which matrix compound is
(i) an organic polymer, and/or
(ii) a silicon-based inorganic adsorbent.

Further, the present invention relates to a process for preparing such a solid dispersion.

Naloxegol oxalate and naloxegol phosphate, both in crystalline form, are the only solid forms of naloxegol which have been described to be suitable for pharmaceutical formulation in WO 2012/044243A1. Surprisingly the present inventors provide naloxegol salts which have been described to be unstable and to delinquesce, as stable solid material suitable for pharmaceutical formulation. Briefly, a stable solid dispersion comprising naloxegol in amorphous form can be provided when combining the naloxegol with at least one matrix compound. This surprisingly stable solid dispersion is advantageous with respect to its suitability for pharmaceutical formulation when compared to the solid forms described for the corresponding naloxegol salts described in WO 2012/044243A1. Further, the solid dispersion of the present invention shows advantageous long term stability in comparison with the corresponding naloxegol salts described in WO 2012/044243A1.

### Amorphous

As described above, the solid dispersion according to the present invention comprises naloxegol salt in amorphous form, and at least one matrix compound.

"Amorphous" in the context of the invention means that the solid phase is in a non-crystalline state. Amorphous solids generally possess no long-range order of molecular packing. The solid state form of a solid active pharmaceutical ingredient, such as of the µ-Opioid antagonist naloxegol in the solid dispersion may be determined by polarized light microscopy, X-ray powder diffraction, differential scanning calorimetry or other techniques known to those of skill in the art. The term "comprising" an "active pharmaceutical ingredient", such as naloxegol or a salt thereof, in amorphous form means that at least 80 % by weight, more preferably at least 85 % by weight, more preferably at least 90 % by weight, more preferably at least 95 % by weight, more preferably at least 96 % by weight, more preferably at least 97 %, more preferably at least 98 % by weight, more preferably at least by weight 99 % by weight, more preferably at least by weight 99.9 % by weight, more preferably all of the active pharmaceutical ingredient, such as naloxegol or a salt thereof, present in the solid dispersion is present in amorphous form.

Besides the amorphous form of the active pharmaceutical ingredient, such as naloxegol or a salt thereof, the solid dispersion may comprise crystalline forms of the active pharmaceutical ingredient, such as naloxegol or a salt thereof. However, preferably, less than 20 % by weight, more preferably less than 15 % by weight, more preferably less than 10 % by weight, more preferably less than 5 % by weight, more preferably less than 4 % by weight, more preferably less than 3 %, more preferably less than 2 % by weight, more preferably less than 1 % by weight, more preferably less than 0.1 % by weight, more preferably 0 % by weight of all the active pharmaceutical ingredient, such as naloxegol or a salt thereof, present in the solid dispersion is present in crystalline form. Preferably the solid dispersion thus does not comprise any detectable active pharmaceutical ingredient, such as naloxegol or a salt thereof, in crystalline form.

Thus, the present invention preferably relates to a solid dispersion, as described above, wherein at least 80 % by weight, more preferably at least 85 % by weight, more preferably at least 90 % by weight, more preferably at least 95 % by weight, more preferably at least 96 % by weight, more preferably at least 97 %, more preferably at least 98 % by weight, more preferably at least by weight 99 % by weight, more preferably at least by weight 99.9 % by weight, more preferably all of the active pharmaceutical ingredient, such as naloxegol or a salt thereof, present in the solid dispersion is present in amorphous form. Likewise, the present invention relates to a process for preparing a solid dispersion, as described above, wherein at least 80 % by weight, more preferably at least 85 % by weight, more preferably at least 90 % by weight, more preferably at least 95 % by weight, more preferably at least 96 % by weight, more preferably at least 97 %, more preferably at least 98 % by weight, more preferably at least by weight 99 % by weight, more preferably at least by weight 99.9 % by weight, more preferably all of the active pharmaceutical ingredient, such as naloxegol or a salt thereof, present in the solid dispersion is present in amorphous form.

### Solid dispersion

The term "solid dispersion" refers to a composition in a solid state, i.e. a state which is neither liquid nor gaseous, wherein the active pharmaceutical ingredient, such as naloxegol or a salt thereof, is dispersed in at least one of the at least one pharmaceutically acceptable matrix compounds comprised in the solid dispersion, preferably in all of the pharmaceutically acceptable matrix compounds comprised in the solid dispersion.

Preferably the solid dispersion consist of the active pharmaceutical ingredient, such as naloxegol or a salt thereof, and the at least one matrix compound. Preferably, the at least one matrix compound forms a matrix which may be either crystalline or amorphous or a mixture thereof. The active pharmaceutical ingredient, such as naloxegol or a salt thereof, can in principle be dispersed in the matrix molecularly, in purely amorphous particles or in amorphous and crystalline particles, as described above.

The term "solid dispersion" as used herein encompasses all known categories of solid dispersions, *e.g*. simple eutectic mixtures, solid solutions, such as continuous solid solutions, discontinued solid solutions, substitutional crystalline, interstitial crystalline and amorphous solid solutions, glass solutions and amorphous precipitations in crystalline carriers. Preferably, the solid dispersion according to the present invention is an amorphous solid solution.

Preferably, the solid dispersion according to the invention is an amorphous solid dispersion. The term "amorphous solid dispersion" as used herein refers to solid dispersions comprising the active pharmaceutical ingredient, such as naloxegol or a salt thereof, in a substantially amorphous solid form. Preferably, amorphous particles of the active pharmaceutical ingredient, such as naloxegol or a naloxegol salt, are dispersed in the polymer matrix.

The term "substantially amorphous solid form" means that at least 80 % by weight, more preferably at least 85 % by weight, more preferably at least 90 % by weight, more preferably at least 95 % by weight, more preferably at least 96 % by weight, more preferably at least 97 %, more preferably at least 98 % by weight, more preferably at least by weight 99 % by weight, more preferably at least by weight 99.9 % by weight, more preferably all of the active pharmaceutical ingredient, such as naloxegol or the naloxegol salt, is present in amorphous form.

### The matrix component

Regarding the at least one pharmaceutically acceptable matrix compound, this at least one matrix compound is either a polymer or a silicon-based inorganic adsorbent, as mentioned above. It is to be understood that the solid dispersion may comprise more than one matrix compound, such as two, three, four, five, six, seven, eight, nine or ten matrix compounds. In case, more than one matrix compound is present, a mixture of at least one polymer and at least one silicon-based inorganic adsorbent may also be present. Preferably, the solid dispersion comprises one matrix compound.

### The polymer

According to one preferred embodiment (embodiment (i)), the at least one matrix compound is at least one polymer. Preferably, the solid dispersion comprises only one matrix component which is a polymer.

According to this embodiment, the solid dispersion contains the active pharmaceutical ingredient, such as naloxegol or the naloxegol salt, in an amount of at least 10 weight-% based on the combined weight of the active pharmaceutical ingredient (such as naloxegol or naloxegol salt) and the at least one matrix compound. Preferably, the solid dispersion contains the active pharmaceutical ingredient, such as naloxegol or naloxegol salt, in an amount of at least 15 weight-%, preferably at least 20 % by weight, preferably at least 25 % by weight, most preferably in the range of from 10 % to 50 % by weight, based on the combined weight of the active pharmaceutical ingredient (such as naloxegol or naloxegol salt) and the at least one matrix compound.

Polymers which are suitable for use in the dispersion of the present invention are preferably polymers which are pharmaceutically acceptable, in particular for oral administrations, to a mammal such as a human. Preferably, the polymer is a polymer selected from the group consisting of celluloses, hydroxyalkylcellulose, polyethyleneglycols, polyvinylalcohols, vinylpyrrolidone/vinylacetate copolymers, polyvinyl caprolactam/polyvinyl acetate/polyethylene glycol copolymers and mixtures and copolymers thereof.

As suitable examples for celluloses, cellulose acetate phthalates, carboxymethyl celluloses and carboxymethyl celluloses and carboxyethyl celluloses are mentioned. It is to be understood that this includes derivatives of the above-mentioned polymers such as chemically modified derivatives thereof. In case the polymer is a hydroxyalkyl cellulose, the polymer is preferably a hydroxymethyl cellulose, a hydroxypropyl cellulose, a hydroxypropyplmethyl cellulose or a hydroxymethyl cellulose acetate succinate. Suitable examples for celluloses as such are cellulose acetate phthalates, carboxymethyl cellulose and carboxyethyl cellulose. The term "cellulose acetate phthalate" refers to any of the family of cellulose polymers that have acetate and phthalate groups attached by ester linkage to a significant fraction of the cellulose polymer's hydroxyl groups.

Preferably, the polymer is selected from the group consisting of hydroxymethyl cellulose, hydroxypropyl cellulose, hydroxypropylmethyl cellulose, hydroxymethyl cellulose acetate succinate, hydroxypropylmethyl cellulose acetate succinate, hydroxypropylmethyl cellulose acetate phthalate, hydroxypropylmethyl cellulose acetate, hydroxypropylmethyl cellulose succinate, hydroxypropylmethyl cellulose phthalate, hydroxypropylmethyl cellulose, hydroxypropyl cellulose, methyl cellulose, hydroxyethyl cellulose, hydroxyethylmethyl cellulose, hydroxyethyl cellulose acetate, hydroxyethylethyl cellulose, hydroxyethylmethyl cellulose acetate succinate, hydroxyethylmethyl cellulose acetate phthalate, carboxymethyl cellulose, carboxyethyl cellulose, polyethyleneglycol, polyvinylalcohol, vinylpyrrolidone/vinylacetate copolymers, polyvinylcaprolactam polyvinylacetate polyethyleneglycol copolymers and mixtures or copolymers thereof. In case the polymer is a polyvinylcaprolactam polyvinylacetate polyethyleneglycol copolymer, the polymer is preferably a graft polymer. The term "graft polymer" as used herein refers to a branched copolymer in which the side chains are structurally distinct from the main chain. Preferably, these graft polymers are based on polyethers and are obtained via free radical polymerization of the vinyl monomers in the presence of polyethers. In case of a polyvinylcaprolactam polyvinylacetate polyethyleneglycol graft polymer, a vinylcaprolactam and vinylacetate are used as vinyl monomers. Graft polymers of this type are commercially available as Soluplus^{®}, BASF. More preferably, the polymer is selected from the group consisting of hydroxymethyl cellulose, hydroxypropyl cellulose, polyethylene glycol, polyvinyl alcohol, hydroxypropylmethyl cellulose, hydroxymethyl cellulose acetate succinate, vinylpyrrolidone/vinylacetate copolymers and polyvinylcaprolactam polyvinylacetate polyethylene glycol graft polymers.

Thus, the present invention also relates to a solid dispersion, as described above, wherein the polymer is selected from the group consisting of hydroxymethyl cellulose, polyvinylpyrrolidone, hydroxypropyl cellulose, polyethylene glycol, polyvinyl alcohol, hydroxypropylmethyl cellulose, hydroxypropylmethyl cellulose acetate succinate, vinylpyrrolidone/vinylacetate copolymers and polyvinylcaprolactam polyvinylacetate polyethylene glycol graft polymers, wherein preferably at least 80 % by weight of all of the active pharmaceutical ingredient, such as naloxegol or naloxegol salt, present in the solid dispersion is present in amorphous form.

Most preferably, the polymer is a hydroxyalkylalkylcellulose or a polyvinylcaprolactam-polyvinylacetate-polyethylene glycol graft polymer. Preferably, the weight average molecular weight (M_{w}) of the hydroxyalkylalkylcellulose is 6kDa to 600 kDa. According to the present invention, it is possible that the solid dispersion contains two or more cellulose derivative, preferably two or more hydroxyalkylalkylcelluloses, more preferably two or more hydroxypropylmethylcelluloses (HPMC) which differ only in the weight average molecular weight M_{w}.

Preferably, the molecular degree of substitution (DS) of the hydroxyalkylalkylcellulose, more preferably the hydroxypropylmethylcellulose (HPMC), is in the range of from 0.05 to 2.8, preferably in the range of from 0.1 to 2.5, more preferably in the range of from 0.2 to 2.3, more preferably in the range of from 0.2 to 2.2. According to the present invention, it is possible that the solid dispersion contains two or more cellulose derivative, preferably two or more hydroxyalkylalkylcelluloses, more preferably two or more hydroxypropylmethylcelluloses which differ only in the molecular degree of substitution. The parameter DS describes the number of hydroxyalkylalkylated sites per anhydroglucose unit of a given hydroxyalkylalkylcellulose.

More preferably, hydroxyalkylalkylcellulose is hydroxypropylmethylcellulose acetate succinate, where the degree of substitution (DS) of methoxy on hydroxypropylmethylcellulose (HPMC) ranges from 1.78 to 2.02 while the molar substitution of hydroxypropoxy is 0.23 to 0.41. An acetic acid and succinic anhydride are reacted with HPMC under specifically controlled conditions to produce HPMCAS having desired DS. The weight average molecular weight (M_{w}) of HPMC AS is preferably between 10 kDa to 500 kDa.

Likewise, the present invention relates to a process for preparing a solid dispersion and a solid dispersion obtained or obtainable by said process, wherein step b) comprises bringing the solution or suspension of naloxegol free base into contact with a suitable salt-forming acid and followed by step (c), which comprises bringing the solution or suspension obtained from step b) into contact with the at least one acceptable matrix compound. The at least one acceptable matrix compound is a polymer, wherein the polymer is selected from the group consisting of hydroxymethyl cellulose, polyvinylpyrrolidone, hydroxypropyl cellulose, polyethylene glycol, polyvinyl alcohol, hydroxypropylmethyl cellulose, hydroxypropylmethyl cellulose acetate succinate, vinylpyrrolidone/vinylacetate copolymers and polyvinylcaprolactam polyvinylacetate polyethylene glycol graft polymers.

Surprisingly, it has been found that, when using a polymer as described above, naloxegol salt can be stabilized in amorphous form. In other words, a stable solid dispersion comprising naloxegol salt in amorphous form is provided. The term "stable" in this context is denoted to mean that the total amount of naloxegol salt present in amorphous form present in the solid dispersion does not change over a time up to 4 weeks, in particular when exposed to 75 % relative humidity at 40 °C for four weeks.

The at least one polymer forms a matrix which may be either crystalline or amorphous or a mixture thereof. Preferably, the polymer forms a matrix which is amorphous. The term "amorphous" in this context is denoted to mean that at least 80 % by weight, more preferably at least 85 % by weight, more preferably at least 90 % by weight, more preferably at least 95 % by weight, more preferably at least 96 % by weight, more preferably at least 97 %, more preferably at least 98 % by weight, more preferably at least by weight 99 % by weight, more preferably at least by weight 99.9 % by weight, more preferably all of the polymer(s) present in the solid dispersion is/are present in amorphous form.

### The silicon-based inorganic adsorbent

According to one embodiment (embodiment (ii)), the at least one matrix compound is at least one silicon-based inorganic adsorbent. Preferably, the solid dispersion comprises only one matrix component which is a silicon-based inorganic adsorbents.

Preferably, the solid dispersion in (ii) contains the active pharmaceutical ingredient, such as naloxegol or the naloxegol salt, in an amount in the range of from 10 to 50 weight-%, more preferably in the range of from 15 to 40 % by weight, more preferably in the range of from 20 % to 35 % by weight based on the combined weight of naloxegol salt and at least one matrix compound.

Examples of silicon-based inorganic adsorbents include, but are not restricted to, silica, silicates, and a combination of two or more thereof. For example, the silicon-based inorganic adsorbent is selected from the group consisting of silicas and combinations of two or more thereof; or from the group consisting of silicates and combinations of two or more thereof; or from the group consisting of at least one silicate. The term "silicate" as used in this context of the present invention refers to naturally occurring or synthesized compounds containing an anionic silicon compound, preferably an oxide. Examples of such silicates include, but are not restricted to, nesosilicates comprising the structure unit [SiO_{4]}⁴⁻, sorosilicates comprising the structure unit [Si₂O₇]⁶⁻, cyclosilicates comprising the structure unit [SiₙO₃ₙ]²ⁿ⁻, single chain inosilicates comprising the structure unit [SiₙO₃ₙ]²ⁿ⁻, double chain inosilicates comprising the structure unit [Si₄nO₁₁ₙ]⁶ⁿ⁻, phyllosilicates comprising the structure unit [SiₙO₅ₙ]²ⁿ⁻, or tectosilicates with a 3D framework comprising the structure unit [AlₓSiyO_{2(x+y)}]^{x-}. The term "silica" as used in this context of the present invention refers to naturally occurring or synthesized silica. Examples of such silica include, but are not restricted to fumed silica, precipitated silica, gel silica, colloidal silica.

Preferably the silicon-based inorganic adsorbent has a pH of at least 6.0. More preferably, the silicon-based inorganic adsorbent has a pH in the range of from 6.0 to 9.0, preferably in the range of from 6.5 to 8.5, more preferably in the range of from 6.8 to 8.0. The pH has been determined by suspending 2 g of the respective adsorbent in 50 ml water. After stirring the suspension was allowed to stand for two minutes and the pH was determined with a pH meter at room temperature.

Generally, it is conceivable that the solid dispersion of the present invention contains at least one silicon-based inorganic adsorbent having a pH in the above-defined preferred ranges and at least one silicon-based inorganic adsorbent having a pH outside these ranges. Preferably, all silicon-based inorganic adsorbents comprised in the solid dispersion of the present invention have a pH in the above-defined preferred ranges.

Preferably, the oil adsorbance of the at least one silicon-based inorganic adsorbent is in the range of from 1.0 to 5.0 ml/g, preferably in the range of from 1.3 to 4.5 ml/g, more preferably in the range of from 1.5 to 4.0 ml/g, more preferably in the range of from 2 to 3.5 ml/g. Generally, it is conceivable that the solid dispersion of the present invention contains at least one silicon-based inorganic adsorbent having an oil adsorbance in the above-defined preferred ranges and at least one silicon-based inorganic adsorbent having an oil adsorbance outside these ranges. Preferably, all silicon-based inorganic adsorbents comprised in the solid dispersion of the present invention have an oil adsorbance in the above-defined preferred ranges.

Preferably, the bulk density of the at least one silicon-based inorganic adsorbent is in the range of from 0.05 to 0.25 g/ml, preferably in the range of from 0.10 to 0.16 g/ml, more preferably in the range of from 0.10 to 0.16 g/ml Generally, it is conceivable that the solid dispersion of the present invention contains at least one silicon-based inorganic adsorbent having a bulk density in the above-defined preferred ranges and at least one silicon-based inorganic adsorbent having a bulk density outside these ranges. Preferably, all silicon-based inorganic adsorbents comprised in the solid dispersion of the present invention have having a bulk density in the above-defined preferred ranges.

Preferably, the silica is selected from the group consisting of fumed silica, precipitated silica, gel silica, colloidal silica, and a combination of two or more thereof, such as a combination of fumed silica and precipitated silica or a combination of fumed silica and colloidal silica or a combination of fumed silica and gel silica or a combination of precipitated silica and gel silica or a combination of precipitated silica and colloidal silica or a combination of gel silica and colloidal silica or a combination of fumed silica and precipitated silica and gel silica or a combination or fumed silica and gel silica and colloidal silica or a combination of precipitated silica and gel silica and colloidal silica or a combination of fumed silica and precipitated silica and gel silica and colloidal silica. Preferred silica include, but are not restricted to, the commercially available compounds Syloid® 72 FP, Syloid® 244 FP, both from Grace.

Preferably, the silicate is an aluminosilicate which, more preferably, additionally contains at least one alkali metal element selected from the group consisting of Li, Na, K, Rb, Cs and a combination of two or more thereof, preferably from the group consisting of Li, Na, K, and a combination of two or more thereof, more preferably from the group consisting of Na, K, and a combination of two or more thereof, and/or at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate which additionally contains at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate which additionally contains Mg. Preferred silicates include, but are not restricted to, the commercially available compounds Neusilin® UFL2, Neusilin® US2, both from Fuji Chemical Industry Co., Ltd.

Therefore, the present invention also relates to the solid dispersion as described above, wherein the at least one silicon-based inorganic adsorbent is selected from the group consisting of silica, silicates, and a combination of two or more thereof, wherein the silica is preferably selected from the group consisting of fumed silica, precipitated silica, gel silica, colloidal silica, and a combination of two or more thereof, and wherein the silicates are preferably aluminosilicates preferably comprising at least one alkali metal element and/or at least one alkaline earth metal element, more preferably at least one alkaline earth metal element, more preferably magnesium.

Thus, the present invention also relates to solid dispersion, as described above, and to a process for preparing a solid dispersion, as described above, wherein the at least one matrix compound is at least one silicon-based inorganic adsorbent, and wherein the at least one silicon-based inorganic adsorbent is selected from the group consisting of silica, silicates, and a combination of two or more thereof, wherein the silica is preferably selected from the group consisting of fumed silica, precipitated silica, gel silica, colloidal silica, and a combination of two or more thereof, and wherein the silicates are preferably aluminosilicates preferably comprising at least one alkali metal element and/or at least one alkaline earth metal element, more preferably at least one alkaline earth metal element, more preferably magnesium, wherein more preferably, at least 90 weight-%, more preferably at least 95 weight-%, more preferably at least 99 weight-% of the at least one silicon-based inorganic adsorbent are present in amorphous form.

Generally, the silica and/or the silicate can be present in crystalline or amorphous form. Preferably, at least 90 weight-%, more preferably at least 95 weight-%, more preferably at least 99 weight-% of the at least one silicon-based inorganic adsorbent are present in amorphous form. More preferably, at least 99.5 weight-%, more preferably at least 99.9 weight-%, more preferably at least 99.99 weight-% of the at least one silicon-based inorganic adsorbent are present in amorphous form.

According to this embodiment, the weight %ages for the silicon-based inorganic adsorbent are calculated based on the combined weight of the naloxegol salt and at least one matrix compound.

### Pharmaceutically acceptable salts

As described above, the active pharmaceutical ingredient, such as naloxegol, can be present in the form of a pharmaceutically acceptable salt. The term "pharmaceutically acceptable salts" refers to salts prepared from pharmaceutically acceptable, preferably non-toxic, bases or acids including mineral or organic acids or organic or inorganic bases. Such salts are also known as acid addition and base addition salts, with naloxegol acid addition salts being preferred. Acids commonly employed for the preparation of acid addition salts of an active pharmaceutical ingredient, such as naloxegol, are inorganic acids such as hydrochloric acid, hydrobromic acid, hydroiodic acid, sulfuric acid, phosphoric acid and the like, and organic acids such as para-toluene sulfonic acid, methane sulfonic acid, oxalic acid, para-bromophenylsulfonic acid, carbonic acid, succinic acid, citric acid, D-tartaric acid, benzoic acid, acidic acid, ethanedisulphonic acid and the like, with tartaric acid and ethanedisulphonic acid being preferred.

Examples of pharmaceutically acceptable salts are sulfates, pyrosulfates, bisulfates, sulfites, bisulfites, phosphates, monohydrogenphosphates, dihydrogenphosphates, metaphosphates, pyrophosphates, bromides, iodides, acetates, propionates, dicanoates, caprolates, acrylates, formates hydrochlorides, dihydrochlorides, isobutyrates, caproates, heptanoates, propiolates, oxalates, malonates, succinates, suberates, sebacates, fumarates, maleates, butin-1,4-dioates, hexin-1,6-dioates, benzoates, chlorobenzoates, methylbenzoates, hydroxybenzoates, methoxybenzoates, phthalates, xylenesulfates, phenylacetates, phenylpropionates, phenylbutyrates, citrates, lactates, gamma-hydroxybutyrrates, glycolates, tartrates, methanesulfonates, propanesulfonates, naphthalene-1-sulfonates, naphthalene-2-sulfonates, mandelates and salts derived from other primary, secondary or tertiary amines including amines such as arginines, betaines, caffeines, cholines, N,N'-dibenzylethylenediamines, diethyleneethylamines, 2-diethylaminoethanols, 2-dimethylaminoethanols, ethanolamines, ethylenediamines, N-ethylmorpholines, N-ethylpiperidines, cocamines, glucosamines, histidines, hydrabamines, isopropylamines, lysines, methylglucamines, morpholines, piperazines, piperidines, polyamine resins, purines, thiobromines, triethylamines, trimethylamines, tripropylamines, tromethamines and the like. Preferred pharmaceutically acceptable acid addition salts of naloxegol are those formed with mineral acids such as hydrochloric acid and hydrobromic acid and those formed with organic acids such as D-tartaric acid, ethanedisulphonic acid and methanesulfonic acid.

Preferably the naloxegol salt is a salt of naloxegol with an inorganic acid. Preferably the inorganic acid is characterized by having at least one acidic group with a pKa of at most 4.0, with phosphoric acid or hydrochloric acid being particularly preferred.

Alternatively the naloxegol salt is a salt of naloxegol with an organic acid. Preferably the organic acid contains from 2 to 8 carbon atoms. More preferably in the solid dispersion, the organic acid containing from 2 to 8 carbon atoms comprises a carboxyl group. Alternatively in solid dispersion, the organic acid containing from 2 to 8 carbon atoms comprises a carboxyl group, with dicarboxylic acids, such as tartaric acid, being particularly preferred.
Preferably the organic acid may comprise a sulfonic acid group, with organic disulfonic acids, such as 1,2-ethanedisulfonic acid, being preferred. A preferred naloxegol salt in a naloxegol solid dispersion is a hemi-1,2-ethanedisulfonate salt.

Besides the above-mentioned components, *i.e*. the pharmaceutically acceptable salt of an active pharmaceutical ingredient, such as a pharmaceutically acceptable naloxegol salt, and the pharmaceutically acceptable matrix, the solid dispersion may comprise further components such as further polymers, optionally at least one solvent, and the like.

Preferably, the solid dispersion comprises no further µ-opioid antagonists besides naloxegol and its pharmaceutical acceptable salts.

As describe above, the solid dispersion may comprise at least one solvent, such as the solvent used in the process for preparing the solid dispersion. In case the solid dispersion comprises at least one solvent, the amount of solvent present in the solid dispersion is preferably less than 5 % by weight, more preferably less than 4 % by weight, more preferably less than 3 % by weight, more preferably less than 2 % by weight, more preferably less than 1 % by weight and most preferably less than 0.5 % by weight, based on the total weight of the solid dispersion.

Preferably, the solid dispersion according to the invention consists of a pharmaceutically acceptable salt of an active pharmaceutical ingredient, such as a naloxegol salt, and at least one pharmaceutically acceptable matrix compound, optionally the at least one solvent, wherein the solvent is preferably present in an amount of less than 5 % by weight, more preferably less than 4 % by weight, more preferably less than 3 % by weight, more preferably less than 2 % by weight, more preferably less than 1 % by weight, and most preferably less than 0.5 % by weight, based on the total weight of the solid dispersion. More preferably, the solid dispersion as described above consists of the pharmaceutically acceptable salt of an active pharmaceutical ingredient, such as naloxegol or salt thereof, and the polymer.

### Process for preparing the solid dispersion

As described above, the present invention also relates to a process for preparing a solid dispersion comprising a pharmaceutically acceptable salt of an active pharmaceutical ingredient, such as a naloxegol salt, in amorphous form and at least one pharmaceutically acceptable matrix compound, the process comprising
a) providing the active pharmaceutical ingredient, such as naloxegol, in a solvent,
b) bringing the solution or suspension provided in a step a) into contact with a suitable salt-forming acid,
c) bringing the solution or suspension obtained from step b) into contact with the at least one acceptable matrix compound,
d) removing at least part of the solvent to provide the solid dispersion comprising a salt of a pharmaceutically active ingredient, such as a naloxegol salt, and at least one pharmaceutical acceptable matrix compound.

### Step (a)

The provision of naloxegol may be carried out by any method known to the person skilled in the art, such as, e.g., the methods described in WO 2005/058367 A2, for example as described on page 62.

Naloxegol may be provided in any form, such as in crystalline or in amorphous form. Preferably, naloxegol is provided as described in WO 2005/058367 A2 on page 62.

Preferably, the naloxegol is dissolved in at least one solvent. Regarding the at least one solvent, no specific restrictions exist. Preferably, the at least one solvent is selected from the group consisting of C1-C3 ketones, C1-C2 halogenated hydrocarbons, C1-C4 alcohols, C2-C6 ethers, C3-C5 esters, and a combination of two or more thereof, more preferably from the group consisting of C3-C5 esters, more preferably ethylacetate. The skilled person will be able to provide other active pharmaceutical ingredients in an analogous manner.

### Step (b)

In step (b), the active pharmaceutical ingredient, such as naloxegol, provided in step (a) is brought into contact with a suitable salt-forming agent, preferably a suitable salt-forming acid. A salt of the active pharmaceutical ingredient, such as naloxegol salt, forms, which may remain in solution upon step b) or which may form a suspension upon step b).

The suitable salt forming agent, preferably a suitable salt-forming acid, may be added as such to the suspension, or preferably solution, obtained from step a), thereby bringing the active pharmaceutical ingredient, such as naloxegol, and the salt-forming acid into contact. The suitable salt forming agent, preferably a suitable salt-forming acid, may also be added as a solution or suspension of the salt forming agent in a further solvent to the suspension, or preferably solution, obtained from step a), thereby bringing the active pharmaceutical ingredient, such as naloxegol, and the salt-forming acid into contact. It is preferred that the further solvent, wherein the salt-forming agent is dissolved or suspended, is the same solvent as used in step a), and preferably the solvent for both steps a) and b) comprises, and preferably is, ethylacetate. A salt of the active pharmaceutical ingredient, such as naloxegol, with the salt-forming agent, preferably the salt-forming acid, forms upon step b), which salt may be in solution or in suspension.

To the suspension thus obtained solvent or solvent mixtures of hydrocarbons, such as heptane, may be added.

### Step c)

In step c), the suspension or solution obtained from step b) is brought into contact with the at least one pharmaceutically acceptable matrix. The salt of the active pharmaceutical ingredient, such as the naloxegol salt, may be dissolved or dispersed together with the at least one matrix compound in a suitable solvent. Both components may be dissolved or dispersed together or subsequently.

The term "a suitable solvent" as used herein refers to a solvent or solvent mixture in which both the salt of the active pharmaceutical ingredient, such as the naloxegol salt, and the at least one matrix compound have adequate solubility or may be suitably dispersed. The term "adequate solubility" is denoted to mean a solubility at room temperature of greater than about 10 mg/ml. In case the naloxegol salt and the polymer require different solvents to obtain the desired solubility, preferably a mixture of solvents is used. In this case, the salt of the active pharmaceutical ingredient, such as the naloxegol salt, may be dissolved in at least one solvent to give a mixture comprising the at least one solvent and the salt. Likewise, the polymer may be dissolved in at least one further solvent to give a mixture comprising the polymer and the at least one further solvent. Both mixtures may then be mixed together.

The salt of the active pharmaceutical ingredient, such as the naloxegol salt, and polymer may be dissolved or dispersed, together or subsequently, in the suitable solvent (including solvent mixtures).

Examples of suitable solvents for naloxegol salt include, but are not limited to C5-C10 hydrocarbons, C3-C4 alcohols, C2-C6 ethers, C3-C5 esters, and a combination of two or more thereof, more preferably a combination of C5-C10 hydrocarbons and C3-C5 esters.

In this case where the at least one matrix compound is selected from the group consisting of silicon-based inorganic adsorbents and a combination of two or more thereof, it is preferred that the process comprises dispersing the at least one matrix compound in the solution comprising the naloxegol salt.

Consequently, solvents are preferred in which the salt of the active pharmaceutical ingredient, such as the naloxegol salt, can be dissolved and the at least one silicon-based inorganic adsorbent can be dispersed. Preferably for the naloxegol salt, the at least one suitable solvent is selected from the group consisting of C5-C10 hydrocarbons, C3-C4 alcohols, C2-C6 ethers, C3-C5 esters, and a combination of two or more thereof, more preferably a combination of C5-C10 hydrocarbons and C3-C5 esters, and a combination of two or more thereof.

Regarding the weight ratio of the salt of the active pharmaceutical ingredient, such as the naloxegol salt, and the at least one silicon-based inorganic adsorbent relative to the at least one solvent, no specific restrictions exist provided that the finally obtained mixture is a mixture wherein the at least one silicon-based inorganic adsorbent is dispersed in a solution of the salt of the active pharmaceutical ingredient, such as the naloxegol salt, in the at least one solvent, which mixture can be subjected to the subsequent step (d). Preferably, the weight ratio of the salt of the active pharmaceutical ingredient, such as the naloxegol salt, plus the at least one silicon-based inorganic adsorbent, preferably the naloxegol salt plus the at least one silica, relative to the at least one solvent, preferably the mixture of ethylacetate and heptane, is in the range of from 0.01: 1 to 0.3 : 1, preferably in the range of from 0.02 : 1 to 0.2 : 1, more preferably in the range of from 0.05 : 1 to 0.2 : 1.

To accelerate and/or improve the solution process of the salt of the active pharmaceutical ingredient, such as the naloxegol salt, in the at least one solvent, suitable methods can be applied. For example, the solution process can be influenced by choosing suitable temperatures, by stirring, and/or by subjecting the respective mixtures to sonication, wherein these methods can be applied during the entire or one or more parts of the mixing process.

Preferably, the dispersion of the at least one silicon-based inorganic adsorbent, preferably selected from the group consisting of silica, silicates, and a combination of two or more thereof, in the solution of the salt of the active pharmaceutical ingredient, such as the naloxegol salt, in the at least one solvent, is prepared at a temperature in the range of from 10 to 40 °C, more preferably in the range of from 15 to 35 °C, more preferably in the range of from 20 to 30 °C, preferably at ambient pressure.

### Step (d)

In step (d) of the above described method, at least part, preferably essentially all, of the solvent is removed. "Essentially all" is denoted to mean that at least 95 % by weight, more preferably at least 96 % by weight, more preferably at least 97 % by weight, more preferably at least 98 % by weight, more preferably at least 99 % by weight, more preferably at least 99.9 % by weight, more preferably all of the solvent present in the solution according to step (c) is removed in step (d).

Preferably, the solid dispersion obtained or obtainable by this process thus comprises less than 5 % by weight, more preferably less than 4 % by weight, more preferably less than 3 % by weight, more preferably less than 2 % by weight, more preferably less than 1 % by weight, more preferably less than 0.1 % by weight, based on the total weight of the solid dispersion, of the solvent. Most preferably, essentially all of the solvent present in the solution is removed to give the solid dispersion.

The removal of the solvent may be carried out by any suitable method known to those skilled in the art such as filtration, evaporation, lyophilisation, melt extrusion, drum drying, freeze drying or other solvent removal processes. Preferably, the solvent is removed by filtration, if upon step c) a suspension is obtained, or by spray drying or evaporation, if upon step c) a solution is obtained. Spray drying is a process well known to those skilled in the art for preparing solid dispersions. In such a spray drying process, the solution is pumped through an atomizer into a drying chamber thereby removing the solvent to form the solid dispersion. A drying process uses hot gases, such as air, nitrogen, nitrogen-enriched air or argon, to dry the particles. The solution can be atomized by conventional means well known in the art, such as a two-fluid sonication nozzle and a two-fluid non-sonication nozzle.

Preferably, the solvent is removed by evaporation, such as by evaporation under reduced pressure. Preferably, the pressure in step (d) is in the range of from 50 to 450 mbar, more preferably in the range of from 50 to 250 mbar, more preferably in the range of from 50 to 200 mbar.

The temperature during evaporation may be varied or held essentially constant and is preferably in the range of from 20 °C to 40 °C, more preferably in the range of from 25 °C to 40 °C, and most preferably in the range of from 35 °C to 40 °C.

It is preferred that after solvent is removed in step d), the obtained solid dispersion is further milled by conventional milling or grinding means.

The obtained solid dispersion comprising a salt of the active pharmaceutical ingredient, such as a naloxegol salt, in amorphous from and the matrix compound is a flowable, and preferably a free-flowing, composition which is suitable for the manufacture of pharmaceutical formulations, such as pharmaceutical formulations of the amorphous dispersion type, e.g. tablets or capsules comprising the solid dispersion of the present invention.

### Pharmaceutical composition

In another aspect of the invention, the present invention relates to a pharmaceutical composition comprising a solid dispersion as described above or a solid dispersion obtained or obtainable by the above-described method. The pharmaceutical composition preferably comprises the solid dispersion as key ingredient together with at least one further excipient in addition to the at least one matrix compound present in the solid dispersion. The at least one further excipient may be included in the solid dispersion or may be subsequently added to or mixed with the dispersion. Preferably, the pharmaceutical composition comprises no further salt of the active pharmaceutical ingredient, such as no further naloxegol salt, in addition to the salt of the active pharmaceutical ingredient present in the solid dispersion, such as the naloxegol salt present in the solid dispersion. Thus, preferably, at least 80 % by weight, more preferably at least 85 % by weight, more preferably at least 90 % by weight, more preferably at least 95 % by weight , more preferably at least 96 % by weight , more preferably at least 97 %, more preferably at least 98 % by weight, more preferably at least by weight 99 % by weight, more preferably at least by weight 99.9 % by weight, more preferably all of the salt of the active pharmaceutical ingredient present in the pharmaceutical composition, such as the naloxegol salt present in the pharmaceutical composition, is present in amorphous form.

Suitable pharmaceutical excipient include, but are not limited to, carriers, fillers, diluents, lubricants, sweeteners, stabilizing agents, solubilizing agents, antioxidants and preservatives, flavoring agents, binder, colorants, osmotic agents preservatives, buffers, surfactants, granulating and disintegrating agents and combinations thereof. Examples of diluents include, without limitation, calcium carbonate, sodium carbonate, lactose, calcium phosphate, microcrystalline cellulose, mannitol, starch, sodium phosphate or the like. Examples for granulating and disintegrating agents are corn starch, alginic acid, sodium starch glycolate, crospovidone, croscarmellose sodium and the like.
The pharmaceutical composition of the present invention may optionally comprise one or more surfactants, which may be ionic or nonionic surfactants. The surfactants can increase the rate of dissolution by facilitating wetting, thereby increasing the maximum concentration of dissolved drug. The surfactants may also make the dispersion easier to process. Surfactants may also stabilize the amorphous dispersions by inhibiting crystallization or precipitation of the drug by interacting with the dissolved drug by such mechanisms as complexation, formation of inclusion complexes, formation of micelles, and adsorption to the surface of the solid drug. Suitable surfactants include cationic, anionic, and nonionic surfactants.

These include for example fatty acids and alkyl sulfonates; cationic surfactants such as benzalkonium chloride); anionic surfactants, such as dioctyl sodium sulfosuccinate and sodium lauryl sulfate (sodium dodecyl sulfate); sorbitan fatty acid esters; Vitamin E TPGS; polyoxyethylene sorbitan fatty acid esters; polyoxyethylene castor oils and hydrogenated castor oils such as Cremophor RH-40 and Cremopher EL; Liposorb P-20, Capmul POE-0, and natural surfactants such as lecithin and other phospholipids and mono- and diglycerides. Thus, the present invention also relates to a pharmaceutical composition as described above, wherein the composition comprises one or more surfactants selected from the group consisting of anionic surfactants and nonionic surfactants.

Preferably, the pharmaceutical composition comprises one or more surfactants selected from sodium dodecyl sulfate and one or more nonionic surfactants selected from (a) sorbitan fatty acid esters, (b) polyoxyethylene sorbitan fatty acid esters, (c) polyoxyethylene castor oils, (d) polyoxyethylene hydrogenated castor oils, and (e) vitamin E TPGS; and mixtures thereof.

Examples of carriers include, without limitation, solvents, saline, buffered saline, dextrose, water, glycerol, ethanol, propylene glycol, polysorbate 80 (Tween-80™), poly(ethylene)glycol 300 and 400 (PEG 300 and 400), PEGylated castor oil (e.g. Cremophor EL), poloxamer 407 and 188, hydrophobic carriers, fat emulsions, lipids, PEGylated phopholids, polymer matrices, biocompatible polymers, lipospheres, vesicles, particles, and liposomes, or combinations thereof.

Examples of osmotic agents include sodium chloride, glycerol, sorbitol, xylitol, glucose, or combinations thereof.

Binders can include acacia gum, starch, gelatin, sucrose, polyvinylpyrrolidone (Providone), sorbitol, or tragacanth methylcellulose, sodium carboxymethylcellulose, hydroxypropyl methylcellulose, ethylcellulose, or combinations thereof.
Examples of fillers can include calcium phosphate, glycine, lactose, maize-starch, sorbitol, sucrose, or combinations thereof.

Exemplary lubricants include magnesium stearate or other metallic stearates, stearic acid, polyethylene glycol, waxes, oils, silica and colloidal silica, silicon fluid, talc, or combinations thereof.

As flavoring agent those flavoring agents approved by the FDA for use in sweets and pharmaceuticals, foods, candies and beverages or the like are preferred. Preferably, these flavoring agents impart flavors such as grape, cherry, citrus, peach, strawberry, bubble gum, peppermint or others. Peppermint, methyl salicylate, orange flavoring and the like are mentioned by way of examples.

The pharmaceutical composition of the present invention preferably contains a therapeutically effective amount of naloxegol salt. The term "therapeutically effective amount" as used herein refers to an amount of naloxegol present in the solid dispersion or pharmaceutical composition being administered that is sufficient to prevent the Opioid-induced bowel dysfunction.

The pharmaceutical composition of the present invention is preferably administered orally to patients, which include, but is not limited to, mammals, for example humans, in the form of, for example, a tablet, pills, granules or the like. It is also apparent that the pharmaceutical composition of the present invention can be administered with other therapeutic and/or prophylactic agents and/or medications that are not medically incompatible therewith.
The solid dispersion described above may be used in combination with other drugs that are used in the prevention, treatment, control, amelioration, or reduction of risk of the diseases or conditions for which compounds of the present invention are useful. Such other drugs may be administered, by a route and in an amount commonly used therefor, contemporaneously or sequentially with the solid dispersion of the present invention.

### Use

The solid dispersion comprising naloxegol, described above, or the pharmaceutical composition comprising naloxegol described above is useful in a method of antagonizing µ-opioid activity. Thus, the present invention also describes the solid dispersion, described above, or a pharmaceutical composition, as described above for use as antagonists of µ-opioid receptor activity. In particular, the solid dispersion, described above, or the pharmaceutical composition, described above is used for treating, preventing, ameliorating, controlling or reducing the risk of a pain by attaching to 'opioid receptors' in the brain and spinal cord. In particular naloxegol salt compositions using solid dispersions is a medicine used in patients in whom treatment with laxatives has failed. It is well-known that opioids relieve pain by attaching to 'opioid receptors' in the brain and spinal cord. However, these receptors are also found in the gut and when opioids attach to the gut receptors, they reduce the movement of the gut and can cause constipation.

The dosage of naloxegol salt in the compositions of this invention may be varied, however, it is necessary that the amount of naloxegol salt be such that a suitable dosage form is obtained. The dosage regimen will be determined by the attending physician and other clinical factors. It is well known in the medical art that the dosage for anyone patient depends upon many factors including the patient's size, body surface area, age, sex, time and route of administration, general health and other drugs being administered concurrently. Efficacy can be monitored by periodic assessment. Naloxegol may be administered to patients (animals and human) in need of such treatment in dosages that will provide optimal pharmaceutical efficacy. A typical dosage can be, for example, in the range of 6.25 to 50 mg, such as 6.25, 12.5, 25 or 50 mg.

It is remarkable that a flowable, indeed a free-flowing, powder of a solid form which allows the preparation of pharmaceutical formulations could be obtained from a salt of an active pharmaceutical ingredient which salt had not been obtained before as an isolated solid.

A "flowable" powder of a solid form as used herein is a powder having a bulk densitity and tapped density such that the ratio dt/db is less than 1.5, wherein dt = tapped density and db = bulk density. There is no specific lower limit for the ratio dt/db. The ratio dt/db may be higher than 1.05, for instance higher than 1.1.

A "free-flowing" powder of a solid form as used herein has a bulk densitity and tapped density such that the compressibility index as defined by ((dt - db)/dt)*100 percent is less than 20 percent.

Bulk density and tapped density are determined according to Method I of USP29 at 20°C and at 40% relative humidity.

Formulation experts are in need of flowable powders, in particular for the preparation of solid dosage forms, such as tablets or capsules, as sticky or hygroscopic solids at the very least make the job of the formulation expert very difficult.

The present invention therefore enriches the repertoire of the formulation expert by making such salts of an active pharmaceutical ingredient, which cannot as such be prepared as flowable powders - and which are thus not useful or not even available for formulation -, available as a flowable powder which now allows the preparation of a pharmaceutical formulation, in particular a solid formulation.

The present invention therefore also relates to the use of a pharmaceutically acceptable solid matrix compound for the preparation of a salt of an active pharmaceutical ingredient as a flowable powder, wherein said salt of an active pharmaceutical ingredient by itself cannot be prepared as a flowable powder at room temperature and at 50% relative humidity, preferably wherein said salt of an active pharmaceutical ingredient by itself cannot be isolated as a solid at room temperature and at 50% relative humidity.

An "active pharmaceutical ingredient" in this context means an organic drug having a molecular weight of at most 2000 Da, preferably having a molecular weight of at least 80 Da and at most 1000 Da. A preferred "salt of an active pharmaceutical ingredient" in the context of the present invention means a salt of an organic drug having a molecular weight of at least 80 Da and at most 1000 Da, which salt has not been described as having been isolated from an organic solvent in solid form, preferably which salt cannot be isolated from an organic solvent in solid form, in particular not if exposed to the atmosphere at 20°C and 50% relative humidity for at most 5 minutes.

The present invention also relates to a method for the preparation of a flowable powder of a salt of an active pharmaceutical ingredient, wherein said salt of an active pharmaceutical ingredient by itself cannot be prepared as a flowable powder at room temperature and at 50% relative humidity, preferably wherein said salt of an active pharmaceutical ingredient by itself cannot be isolated as a solid at room temperature and at 50% relative humidity, the method comprising the step of forming a solid dispersion comprising said salt of the active pharmaceutical ingredient in amorphous form, and at least one pharmaceutically acceptable solid matrix compound. The pharmaceutically acceptable solid matrix compound is typically (i) an organic polymer, or (ii) a silicon-based inorganic adsorbent. The method is a method where said salt of the active pharmaceutical ingredient is generated in situ and contacted with the pharmaceutically acceptable solid matrix compound without said salt having been isolated from the solvent wherein said salt had been prepared. Thus, since isolation of the salt of the active pharmaceutical ingredient and thus exposure to the atmosphere is not necessary in this method, solid dispersions from salts of an active pharmaceutical ingredient which would otherwise decompose or deliquesce upon exposure to the atmosphere as a consequence of isolation now become accessible.

The present invention also relates to a flowable powder of a salt of an active pharmaceutical ingredient, wherein said salt of an active pharmaceutical ingredient by itself cannot be prepared as a flowable powder at room temperature and at 50% relative humidity, preferably wherein said salt of an active pharmaceutical ingredient by itself cannot be isolated as a solid at room temperature and at 50% relative humidity, which flowable powder is a solid dispersion comprising said salt of the active pharmaceutical in amorphous form, and at least one pharmaceutically acceptable solid matrix compound. The pharmaceutically acceptable solid matrix compound is typically (i) an organic polymer, or (ii) a silicon-based inorganic adsorbent.

The present invention also relates to a free-flowing powder of a salt of an active pharmaceutical ingredient, wherein said salt of an active pharmaceutical ingredient by itself cannot be prepared as a free-flowing powder at room temperature and at 50% relative humidity, preferably wherein said salt of an active pharmaceutical ingredient by itself cannot be isolated as a solid at room temperature and at 50% relative humidity, which free-flowing powder is a solid dispersion comprising said salt of the active pharmaceutical in amorphous form, and at least one pharmaceutically acceptable solid matrix compound. The pharmaceutically acceptable solid matrix compound is typically (i) an organic polymer, or (ii) a silicon-based inorganic adsorbent.

Preferred matrix compounds for the solid dispersion are the matrix compounds described for naloxegol salts above, in particular the matrix compounds described for naloxegol tartaric acid salt and/or for naloxegol 1, 2-ethanedisulfonic acid salt. Preferred methods for the preparation of a solid dispersion of a salt of an active pharmaceutical ingredient, which salt by itself cannot be prepared as a flowable powder at room temperature and at 50% relative humidity, preferably wherein said salt of an active pharmaceutical ingredient by itself cannot be isolated as a solid at room temperature and at 50% relative humidity, are the methods described for naloxegol tartaric acid salt and/or for naloxegol 1,2-ethanedisulfonic acid salt.

The present invention also relates to a solid pharmaceutical composition, such as a tablet or a capsule, comprising a solid dispersion of a salt of an active pharmaceutical ingredient, which salt by itself cannot be prepared as a flowable powder at room temperature and at 50% relative humidity, preferably wherein said salt of an active pharmaceutical ingredient by itself cannot be isolated as a solid at room temperature and at 50% relative humidity, which solid dispersion comprises said salt of the active pharmaceutical in amorphous form, and at least one pharmaceutically acceptable solid matrix compound.

The present invention also relates to the use of a flowable powder of a salt of an active pharmaceutical ingredient, which salt of an active pharmaceutical ingredient by itself cannot be prepared as a free-flowing powder at room temperature and at 50% relative humidity, preferably wherein said salt of an active pharmaceutical ingredient by itself cannot be isolated as a solid at room temperature and at 50% relative humidity, for the preparation of a solid pharmaceutical composition, such as a tablet or a capsule.

The present invention also relates to the use of a free-flowing powder of a salt of an active pharmaceutical ingredient, which salt of an active pharmaceutical ingredient by itself cannot be prepared as a free-flowing powder at room temperature and at 50% relative humidity, preferably wherein said salt of an active pharmaceutical ingredient by itself cannot be isolated as a solid at room temperature and at 50% relative humidity, which free-flowing powder is a solid dispersion comprising said salt of the active pharmaceutical in amorphous form, and at least one pharmaceutically acceptable solid matrix compound, for the preparation of a solid pharmaceutical composition, such as a tablet or a capsule.

Preferred pharmaceutical compositions are analogous to the pharmaceutical compositions described for naloxegol salts above, in particular analogous to the pharmaceutical compositions described for naloxegol tartaric acid salt and/or for naloxegol 1,2-ethanedisulfonic acid salt. "Analogous" in this context means that a "naloxegol salt" is to be replaced by "salt of an active pharmaceutical ingredient, which salt of an active pharmaceutical ingredient by itself cannot be prepared as a free-flowing powder at room temperature and at 50% relative humidity, preferably wherein said salt of an active pharmaceutical ingredient by itself cannot be isolated as a solid at room temperature and at 50% relative humidity".

By way of example, the following particularly preferred embodiments of the invention are mentioned:
1. A solid dispersion comprising a naloxegol salt [(5α,6α)-17-allyl-6-(2,5,8,11,14,17,20-heptaoxadocosan-22-yloxy)-4,5-epoxymorphinan-3,14-diol] in amorphous form and at least one pharmaceutically acceptable solid matrix compound.
2. The solid dispersion according to embodiment 1, wherein the at least one matrix compound is
   (i) an organic polymer, or
   (ii) a silicon-based inorganic adsorbent.
3. The solid dispersion according to embodiment 1 or 2, wherein at least 80 % by weight of the naloxegol salt present in the solid dispersion are present in amorphous form.
4. The solid dispersion according to embodiment 1 or 2, wherein at least 90 % by weight of the naloxegol salt present in the solid dispersion are present in amorphous form.
5. The solid dispersion according to embodiment 1 or 2, wherein essentially all of the naloxegol salt present in the solid dispersion is present in amorphous form.
6. The solid dispersion according to any one of embodiments 1 to 5, wherein the naloxegol salt is a salt of naloxegol with an inorganic acid.
7. The solid dispersion according to embodiment 6, wherein the inorganic acid is characterized by having at least one acidic group with a pKa of at most 4.0.
8. The solid dispersion according to embodiments 6 or 7, wherein the inorganic acid is phosphoric acid or hydrochloric acid.
9. The solid dispersion according to any one of embodiments 1 to 5, wherein the naloxegol salt is a salt of naloxegol with an organic acid.
10. The solid dispersion according to embodiment 9, wherein the organic acid contains from 2 to 8 carbon atoms.
11. The solid dispersion according to any one of embodiments 9 or 10, wherein the organic acid comprises a carboxyl group.
12. The solid dispersion according to any one of embodiments 9 to 11, wherein the organic acid is a dicarboxylic acid.
13. The solid dispersion according to any one of embodiments 9 to 12, wherein the organic acid is tartaric acid.
14. The solid dispersion according to any one of embodiments 9 or 10, wherein the organic acid comprises a sulfonic acid group.
15. The solid dispersion according to embodiment 14, wherein the organic acid is a disulfonic acid.
16. The solid dispersion according to embodiment 15, wherein the organic acid is 1,2-ethanedisulfonic acid.
17. The solid dispersion according to embodiment 9 or embodiment 16, wherein the naloxegol salt is a hemi-1,2-ethanedisulfonate salt.
18. The solid dispersion according to any one of embodiments 9 to 13, wherein the organic acid is not oxalic acid.
19. The solid dispersion according to any one of embodiments 2 to 18, wherein the polymer (i) is a cellulose derivative or a polyvinyl caprolactam polyvinyl acetate polyethylene glycol graft polymer.
20. The solid dispersion according to any one of embodiments 2 to 19 wherein the polymer is polyvinyl caprolactam-polyvinyl acetate-polyethylene glycol, preferably soluplus.
21. The solid dispersion according to any one of embodiments 2 to 19, wherein the polymer is a cellulose derivative.
22. The solid dispersion according to embodiment 21, wherein the cellulose derivative is a hydroxyalkylalkylcellulose or a mixture of two or more hydroxyalkylalkylcelluloses.
23. The solid dispersion according to embodiment 22, wherein the hydroxyalkylalkylcellulose is a hydroxypropylmethylcellulose or a hydroxymethylcellulose.
24. The solid dispersion according to embodiment 22, wherein the mixture of two or more hydroxyalkylalkylcelluloses comprises a hydroxypropylmethylcellulose or a hydroxymethylcellulose.
25. The solid dispersion according to any one of embodiments 22 to 24, wherein the hydroxyalkylalkylcellulose is HPMC-AS.
26. The solid dispersion according to any one of embodiments 2 to 18, wherein the at least one silicon-based inorganic adsorbent has an oil adsorbance in the range of from 1.0 to 5.0 ml/g, preferably in the range of from 2 to 3.5 ml/g.
27. The solid dispersion according to any one of embodiments 2 to 18 or 26, wherein the at least one silicon-based inorganic adsorbent has a bulk density in the range of from 0.05 to 0.25 g/ml, preferably in the range of from 0.10 to 0.22 g/ml, more preferably in the range of from 0.10 to 0.16 g/ml.
28. The solid dispersion according to any one of embodiments 2 to 18 or 26 to 27, wherein the at least one silicon-based inorganic adsorbent is selected from the group consisting of silica, silicates, and a combination of two or more thereof, wherein the silica is preferably selected from the group consisting of fumed silica, precipitated silica, gel silica, colloidal silica, and a combination of two or more thereof, and wherein the silicates are preferably aluminosilicates preferably comprising at least one alkali metal element and/or at least one alkaline earth metal element, more preferably at least one alkaline earth metal element, more preferably magnesium, wherein more preferably, at least 90 weight-%, more preferably at least 95 weight-%, more preferably at least 99 weight-% of the at least one silicon-based inorganic adsorbent are present in amorphous form.
29. The solid dispersion according to any one of embodiments 2 to 18 or 26 to 28, wherein the solid dispersion in (ii) contains the naloxegol salt in amorphous form in an amount in the range of from 10 to 70 weight-%, more preferably in the range of from 15 to 50 % by weight, more preferably in the range of from 20 % to 40 % by weight, more preferably in the range of from 20 % to 35 % by weight, based on the combined weight of the naloxegol salt and the at least one matrix compound.
30. A process for the preparation of a solid dispersion comprising a naloxegol salt in amorphous form and at least one pharmaceutical acceptable matrix compound, the process comprising
   a) providing naloxegol in a solvent,
   b) bringing the solution or suspension provided in a step a) into contact with a suitable salt-forming agent, preferably a salt-forming acid,
   c) bringing the solution or suspension obtained from step b) into contact with the at least one acceptable matrix compound,
   d) removing at least part of the solvent to provide the solid dispersion comprising a naloxegol salt and at least one pharmaceutical acceptable matrix compound.
31. The process according to embodiment 30, wherein naloxegol is provided as naloxegol free base.
32. The process according to any one of embodiments 30 and 31, wherein naloxegol is dissolved in the solvent.
33. The process according to any one of embodiments 30 to 32, wherein the solvent is selected from an ester, a nitrile and a ketone.
34. The process according to any one of embodiments 30 to 33, wherein the solvent contains at least 3 and at most 10 carbon atoms.
35. The process according to any one of embodiments 30 to 34, wherein the solvent is ethylacetate.
36. The process according to any one of embodiments 30 to 35, wherein the salt-forming acid in step b) is any one acid according to embodiment 6, embodiment 7, embodiment 8, embodiment 9, embodiment 10, embodiment 11, embodiment 12, embodiment 13, embodiment 14, embodiment 15, embodiment 16 and/or embodiment 17.
37. The process according to any one of embodiments 30 to 36, wherein step b) is effected by adding the salt-forming acid to the solution or suspension provided in a step a).
38. The process of embodiment 37, wherein the salt-forming acid is added as a solution.
39. The process of embodiment 37, wherein the salt-forming acid is added as a suspension.
40. The process of embodiment 37, wherein the salt-forming acid is added as a solid.
41. The process according to any one of embodiments 30 to 40, wherein bringing the solution or suspension obtained from step b) into contact with the at least one acceptable matrix compound is effected by adding the at least one acceptable matrix compound to the solution or suspension obtained from step b).
42. The process according to any one of embodiments 30 to 41, wherein the at least one pharmaceutical acceptable matrix compound is the pharmaceutically acceptable matrix compounds according to embodiment 19, embodiment 20, embodiment 21, embodiment 22, embodiment 23, embodiment 24, embodiment 25, embodiment 26, embodiment 27, embodiment 28 and/or embodiment 29.
43. The process according to any one of embodiments 30 to 42, wherein at least part of the solvent is removed by solvent evaporation.
44. The process of embodiment 43, wherein solvent evaporation takes place at a reduced pressure in the range of from 50 to 500 mbar.
45. The process according to any one of embodiments 30 to 42, wherein at least part of the solvent is removed by filtration.
46. The process according to any one of embodiments 30 to 45, wherein the the solid dispersion comprising a naloxegol salt and at least one pharmaceutical acceptable matrix compound obtained from step d) is further milled to a powder.
47. A solid dispersion obtained or obtainable by the process according to any one of embodiments 30 to 46.
48. A pharmaceutical composition comprising a solid dispersion according to any one of embodiments 1 to 29 or 47.
49. The pharmaceutical composition according to embodiment 48, wherein the composition comprises one or more surfactants selected from the group consisting of anionic surfactants and nonionic surfactants.
50. The pharmaceutical according to embodiments 48 or 49, wherein the composition comprises one or more surfactants selected from sodium dodecyl sulfate and one or more nonionic surfactants selected from (a) sorbitan fatty acid esters, (b) polyoxyethylene sorbitan fatty acid esters, (c) polyoxyethylene castor oils, (d) polyoxyethylene hydrogenated castor oils, and (e) vitamin E TPGS; and mixtures thereof.
51. The pharmaceutical composition according to any one of embodiments 48 to 50 for the treatment of bowel dysfunction.
52. Use of a pharmaceutically acceptable solid matrix compound for stabilizing a naloxegol salt in a solid state, wherein said naloxegol salt by itself deliquesces at room temperature and at 50% relative humidity.
53. Use of a pharmaceutically acceptable solid matrix compound for the preparation of a free-flowing powder comprising a naloxegol salt, wherein said naloxegol salt by itself cannot be prepared as a free-flowing powder at room temperature and at 50% relative humidity, preferably wherein said salt of an active pharmaceutical ingredient by itself cannot be isolated as a solid at room temperature and at 50% relative humidity.
54. Use according to any one of embodiments 52 or 53, wherein said pharmaceutically acceptable solid matrix compound is the pharmaceutically acceptable matrix compounds according to embodiment 19, embodiment 20, embodiment 21, embodiment 22, embodiment 23, embodiment 24, embodiment 25, embodiment 26, embodiment 27, embodiment 28 and/or embodiment 29.
55. Use of a pharmaceutically acceptable solid matrix compound for the preparation of a flowable powder of a salt of an active pharmaceutical ingredient, which salt of an active pharmaceutical ingredient by itself cannot be prepared as a flowable powder at room temperature and at 50% relative humidity, preferably wherein said salt of an active pharmaceutical ingredient by itself cannot be isolated as a solid at room temperature and at 50% relative humidity.
56. Use of a pharmaceutically acceptable solid matrix compound for the preparation of a flowable, preferably free-flowing, powder comprising a salt of an active pharmaceutical ingredient in a solid state, wherein said salt of an active pharmaceutical ingredient by itself cannot be isolated in a solid state at room temperature and at 50% relative humidity.
57. A solid dispersion according to any one of embodiments 1 to 5 and 19-29, wherein any reference to "naloxegol salt" is to be replaced by "a salt of an active pharmaceutical ingredient, which salt of an active pharmaceutical ingredient by itself cannot be prepared as a flowable powder at room temperature and at 50% relative humidity, preferably wherein said salt of an active pharmaceutical ingredient by itself cannot be isolated as a solid at room temperature and at 50% relative humidity".
58. A process according to any one of embodiments 30 to 46, wherein any reference to "naloxegol" is to be replaced by "the active pharmaceutical ingredient" and any reference to "naloxegol salt" is to be replaced by "a salt of an active pharmaceutical ingredient, which salt of an active pharmaceutical ingredient by itself cannot be prepared as a flowable powder at room temperature and at 50% relative humidity, preferably wherein said salt of an active pharmaceutical ingredient by itself cannot be isolated as a solid at room temperature and at 50% relative humidity".
59. A pharmaceutical composition comprising a solid dispersion according to embodiment 57.
60. The pharmaceutical composition according to embodiment 59, wherein the composition comprises one or more surfactants selected from the group consisting of anionic surfactants and nonionic surfactants.
61. The pharmaceutical according to embodiments 59 or 60, wherein the composition comprises one or more surfactants selected from sodium dodecyl sulfate and one or more nonionic surfactants selected from (a) sorbitan fatty acid esters, (b) polyoxyethylene sorbitan fatty acid esters, (c) polyoxyethylene castor oils, (d) polyoxyethylene hydrogenated castor oils, and (e) vitamin E TPGS; and mixtures thereof.
62. A process for the preparation of a solid dispersion comprising a salt of an active pharmaceutical ingredient in amorphous form, which salt of an active pharmaceutical ingredient by itself cannot be prepared as a flowable powder at room temperature and at 50% relative humidity, preferably wherein said salt of an active pharmaceutical ingredient by itself cannot be isolated as a solid at room temperature and at 50% relative humidity,
   and at least one pharmaceutical acceptable matrix compound, the process comprising
   a) providing the active pharmaceutical ingredient in a solvent,
   b) bringing the solution or suspension provided in a step a) into contact with a suitable salt-forming agent, preferably a salt-forming acid,
   c) bringing the solution or suspension obtained from step b) into contact with the at least one acceptable matrix compound,
   d) removing at least part of the solvent to provide the solid dispersion comprising an amorphous salt of the active pharmaceutical ingredient and at least one pharmaceutical acceptable matrix compound.
63. The process according to embodiment 62, wherein the active pharmaceutical ingredient is provided as a non-salt form.
64. The process according to any one of embodiments 62 or 63, wherein said salt of an active pharmaceutical ingredient is not separated from solvent.
65. The process according to any one of embodiments 62 to 64, wherein said salt of an active pharmaceutical ingredient is not exposed to the atmosphere.
66. The process according to any one of embodiments 62 to 65, wherein the obtained solid dispersion comprising said amorphous salt of the active pharmaceutical ingredient and at least one pharmaceutical acceptable matrix compound is subsequently dried to give a flowable powder.

The present invention is further illustrated by the following examples. The examples are not to be construed to be in any way limiting for the scope of the claims and invention.

### EXAMPLES

### Example 1

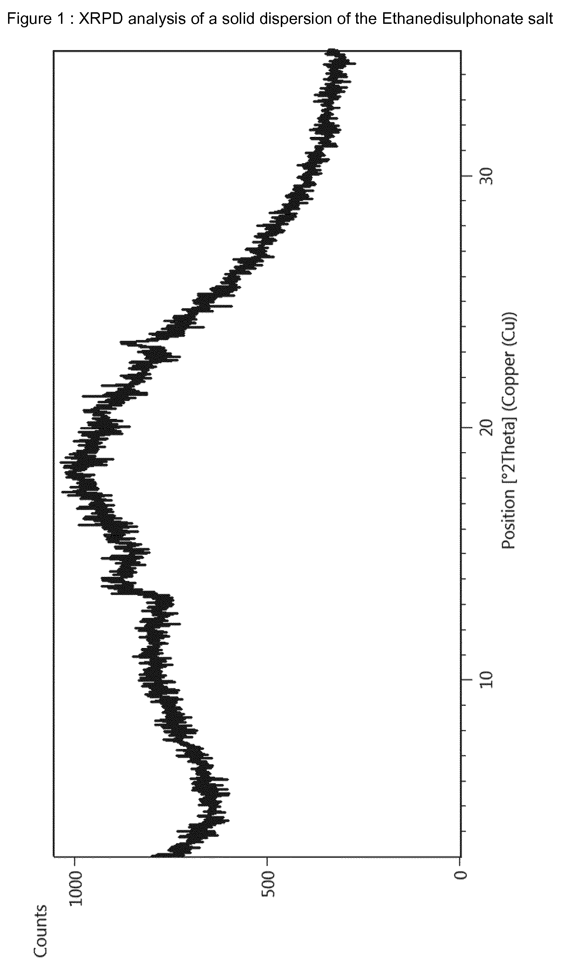

Naloxegol free base 0.25g was dissolved in 1 mL ethyl acetate. In a separate vial 0.053g (0.5 eq) of ethane disulphonic acid dihydrate were suspended in 1 mL ethyl acetate.
The solution of naloxegol free base was then added to the suspension of ethane disulphonic acid dihydrate, gradually in 0.25mL aliquots. Once the last aliquot was added the system was stirred for approximately 1 h. Heptane was then slowly added in 1 mL aliquots up to 30mL heptane to give a ratio of 6:94 (v/v) ethyl acetate to heptane. The resulting mobile slurry was stirred for about 2h. HPMCAS HG polymer (0.855g) was then added to the slurry in 0.2g portions, allowing a time of 30 min between additions. Once the total amount was added, the slurry was stirred for an additional hour before filtration. The resulting solid was dried under vacuum at ambient temperature and ground to produce a free-flowing amorphous solid dispersion, as confirmed by XRD (figure 1).

### Example 2:

Naloxegol free base 0.25g was dissolved in 1 mL ethyl acetate. In a separate vial 0.042g (0.5 eq) of D-tartaric acid was suspended in 1 mL ethyl acetate.
The solution of naloxegol free base was then added to the suspension of D-tartaric acid gradually in 0.25mL aliquots. Once the last aliquot was added the system was stirred for 1 h approx. Heptane was then slowly added in 1 mL aliquots up to 30mL heptane to give a ratio of 6:94 ethyl acetate to heptane v/v%. The resulting mobile slurry was stirred for about 2h. HPMCAS HG polymer (0.855g) was then added to the slurry in 0.2g portions with a time of 30 min between additions. Once the total amount was added, the slurry was stirred for an additional hour before filtration. Drying of the resulting solid under vacuum at ambient temperature gave a free flowing powder. The powder was then ground and a solid dispersion was obtained. The XRD confirmed that the solid dispersion was amorphous.

### Example 3:

Naloxegol free base 0.25g was dissolved in 1 mL ethyl acetate. In a separate vial 0.053g (0.5 eq) of ethane disulphonic acid dihydrate was suspended in 1 mL ethyl acetate.
The solution of naloxegol free base was then added to the suspension of ethane disulphonic acid dihydrate, in a single aliquot. The system was then stirred for 1h approx. HPMCAS HG polymer (0.855g) was then added to the slurry in a single portion. The resulting mixture was then evaporated in a stream of nitrogen to produce a hard, slightly sticky solid mass. The material could be broken up, ground and dried. Drying under vacuum produced a slightly sticky, but flowable solid. The XRD confirmed that the material was substantially amorphous.

## Claims

1. A solid dispersion comprising a naloxegol salt [(5α,6α)-17-allyl-6-(2,5,8,11,14,17,20-heptaoxadocosan-22-yloxy)-4,5-epoxymorphinan-3,14-diol] in amorphous form and at least one pharmaceutically acceptable solid matrix compound.

2. The solid dispersion according to claim 1, wherein the at least one matrix compound is
(i) an organic polymer, or
(ii) a silicon-based inorganic adsorbent.

3. The solid dispersion according to claim 1 or 2, wherein at least 80 % by weight of the naloxegol salt present in the solid dispersion is in amorphous form.

4. The solid dispersion according to any one of claims 1 to 3, wherein the naloxegol salt is a salt of naloxegol with an organic acid.

5. The solid dispersion according to any one of the claims 1 to 4 wherein the organic acid is selected from the group comprising tartaric acid and 1,2-ethanedisulfonic acid.

6. The solid dispersion according to claim 5, wherein the naloxegol salt is a hemi-1,2-ethanedisulfonate salt.

7. The solid dispersion according to claim 5, wherein the naloxegol salt is a D-tartrate salt.

8. The solid dispersion according to any one of the claims 1 to 7, wherein the polymer (i) is a cellulose derivative or a polyvinyl caprolactam polyvinyl acetate polyethylene glycol graft polymer.

9. The solid dispersion according to claim 8, wherein the cellulose derivative is a hydroxyalkylalkylcellulose or a mixture of two or more hydroxyalkylalkylcelluloses.

10. The solid dispersion according to any one of the claims 8 and 9, wherein the hydroxyalkylalkylcellulose is HPMC-AS.

11. A process for the preparation of a solid dispersion comprising a naloxegol salt in amorphous form and at least one pharmaceutical acceptable matrix compound, the process comprising
a) Providing naloxegol in a solvent,
b) bringing the solution or suspension provided in a step a) into contact with a suitable salt-forming acid,
c) bringing the solution or suspension obtained from step b) into contact with the at least one acceptable matrix compound,
d) removing at least part of the solvent to provide the solid dispersion comprising a naloxegol salt and at least one pharmaceutical acceptable matrix compound.

12. The solid dispersion obtained or obtainable by the process according to claim 11.

13. The pharmaceutical composition according to any one of claims 1 to 12 for the treatment of opiod-induced bowel dysfunction.

14. Use of a pharmaceutically acceptable solid matrix compound for isolating a salt of an active pharmaceutical ingredient as a flowable powder, wherein said salt of said active pharmaceutical ingredient by itself cannot be isolated as a flowable powder at room temperature and at 50% relative humidity.

15. Use of a pharmaceutically acceptable solid matrix compound for the preparation of a free-flowing powder comprising a salt of an active pharmaceutical ingredient in a solid state, wherein said salt of said active pharmaceutical ingredient by itself cannot be prepared as a free-flowing powder at room temperature and at 50% relative humidity, preferably wherein said salt of an active pharmaceutical ingredient by itself cannot be isolated as a solid at room temperature and at 50% relative humidity.
